# EUROPEAN PATENT APPLICATION

(11) **EP 4 495 592 A1**
(43) Date of publication of application: **22.01.2025**
(21) Application number: 23770915.9
(22) Date of filing: 17.03.2023
(51) Int. Cl.: G01N 27/62, G01N 33/53, G01N 33/553

(54) **METHOD FOR ANALYZING BIOMOLECULES PRESENT IN VESICLES**

(30) Priority: 17.03.2022 JP 2022042475
(71) Applicant: SHIMADZU CORPORATION, Kyoto-shi, Kyoto 604-8511 (JP)
(72) Inventor: WATANABE, Makoto, Kyoto-shi, Kyoto 604-8511 (JP); SATO, Taka-Aki, Kyoto-shi, Kyoto 604-8511 (JP); MIYAZAWA, Yuta, Otawara-shi, Tochigi 324-8516 (JP)
(74) Representative: Müller-Boré & Partner Patentanwälte PartG mbB
(86) International application number: PCT/JP2023/010599
(87) International publication number: WO 2023/176963

(57) **Abstract**

Provided is a method of analyzing a biomolecule present at a vesicle, the method comprising: bringing a vesicle into contact with a capturing molecule solidified on a support; bringing a first detection molecule labeled with a first metal particle into contact with the vesicle; bringing a second detection molecule labeled with a second metal particle into contact with the vesicle; and quantifying the first metal particle and the second metal particle bonded to the vesicle by inductively coupled plasma (ICP) mass spectrometry or ICP emission spectroscopy.

## Description

### TECHNICAL FIELD

The present invention relates to a method of analyzing a biomolecule present at a vesicle.

### BACKGROUND ART

Extracellular vesicles are small, so it is difficult to directly detect and quantify the surface antigens with a flow cytometer and the like. To address this problem, a method has been developed to detect and quantify surface antigens after capturing vesicles on beads on which an antibody and the like are solidified. PTL 1 discloses a method that comprises bringing exosomes into a reaction with beads to which an antibody capable of recognizing an extracellular vesicle surface marker is bonded, bringing the resultant into a reaction with an APC-labeled tetraspanin optional antibody, and subjecting the resultant to a flow cytometer to detect and quantify the surface marker on the exosomes. PTLs 2 and 3 disclose methods that comprise metal-labeling a target such as a molecule or a virus in cells and detecting the metallic element by inductively coupled plasma mass spectrometry (ICP-MS) to detect the target. PTL 4 discloses a technique capable of simultaneously quantifying a plurality of surface antigens on exosomes by mass spectrometry.

### CITATION LIST

### PATENT LITERATURE

PTL 1: European Patent Laying-Open No. 3093664
PTL 2: International Patent Laying-Open No. WO 2016/109603
PTL 3: International Patent Laying-Open No. WO 2016/182402
PTL 4: International Patent Laying-Open No. WO 2020/235424

### SUMMARY OF INVENTION

### TECHNICAL PROBLEM

When attempting to fix a vesicle on a capturing agent and detect a surface antigen on the vesicle by means of an antibody, it is difficult to simultaneously quantify a plurality of surface antigens with good sensitivity by the method taught by PTLs 1 to 4. The present invention aims at providing an analysis method capable of simultaneously quantifying a plurality of biomolecules present at vesicles with good sensitivity.

### SOLUTION TO PROBLEM

The present invention relates to a method of analyzing a biomolecule present at a vesicle, the method comprising:
bringing a specimen containing a vesicle into contact with a capturing molecule solidified on a support;
bringing a first detection molecule labeled with a first metal particle into contact with the vesicle;
bringing a second detection molecule labeled with a second metal particle into contact with the vesicle; and
quantifying the first metal particle and the second metal particle bonded to the vesicle by inductively coupled plasma (ICP) mass spectrometry or ICP emission spectroscopy.

### ADVANTAGEOUS EFFECTS OF INVENTION

The present invention makes it possible to simultaneously quantify a plurality of biomolecules present at vesicles with good sensitivity.

### BRIEF DESCRIPTION OF DRAWINGS

Fig. 1 is a graph showing quantification of the amount of CD9 expression on the surface of exosomes in Experiment 1, expressed as the amount of gold ions.
Fig. 2 is a graph showing quantification of the amount of CD81 expression on the surface of exosomes in Experiment 1, expressed as the amount of palladium ions.
Fig. 3 is a graph plotting the amount of three types of metal ions (on the vertical axis) relative to the particle number of exosomes (on the horizontal axis) in Experiment 3.
Fig. 4 is a graph plotting the amount of three types of metal ions (on the vertical axis) relative to the incubation time for the cultured cells (on the horizontal axis) in Experiment 4.
Fig. 5 is a graph plotting the amount of three types of metal ions (on the vertical axis) relative to the serum quantity (on the horizontal axis) in Experiment 5.

### DESCRIPTION OF EMBODIMENTS

### [Method of Analyzing Biomolecule Present in Vesicle]

A method of analyzing a biomolecule present at a vesicle according to an aspect of the present invention comprises:
a step of bringing a vesicle into contact with a capturing molecule solidified on a support;
a step of bringing a first detection molecule labeled with a first metal particle into contact with the vesicle;
a step of bringing a second detection molecule labeled with a second metal particle into contact with the vesicle; and
a step of quantifying the first metal particle and the second metal particle bonded to the vesicle by inductively coupled plasma (ICP) mass spectrometry or ICP emission spectroscopy.

The method according to the present invention is capable of simultaneously quantifying a plurality of biomolecules present at vesicles. Once the profile of the plurality of biomolecules present at the vesicles is revealed, it is also possible to identify the type of the vesicles. Because the method according to the present invention detects and quantifies bonding of the detection molecules to the biomolecules by mass spectrometry, it is possible to analyze the biomolecules present at the vesicles with high sensitivity. In addition, the method according to the present invention is capable of analyzing biomolecules present at vesicles in an amount spanning across a wide dynamic range (1×10⁶ to 1×10¹⁰).

Herein, the vesicles have a pouch-shaped structure surrounded by a membrane. The vesicles include extracellular vesicles, envelope viruses, and the like. An extracellular vesicle is a vesicle of a size of several dozen to several hundred nanometers, containing proteins and transmitters such as nucleic acids and surrounded by a lipid bilayer membrane. It is known that extracellular vesicles are released from cells to pass information to other cells. Extracellular vesicles include exosomes, microvesicles, apoptotic bodies, and the like. The size of exosomes is about 50 nm to 150 nm in diameter, for example; the size of microvesicles is about 100 nm to 1 µm in diameter, for example; and the size of apoptotic bodies is about 1 µm to 5 µm in diameter, for example. In the method according to the present invention, the vesicles are preferably exosomes. Biomolecules contained in exosomes are usable for detection and diagnosis of diseases including cancer and lifestyle-related diseases. Examples of the envelope virus include coronavirus, influenza virus, rubella virus, hepatitis C virus, measles virus, hepatitis B virus, human immunodeficiency virus, herpes simplex virus, and the like. The envelope virus may be a recombinant virus.

The biomolecules present at the vesicles may be any molecules including protein, nucleic acid, carbohydrate chain, and the like. The protein present at the vesicles may be a membrane protein, a cell adhesion molecule, a receptor protein, a protein involved in membrane fusion or dissociation, a major histocompatibility complex (MHC) molecule, a heat-shock protein, a disease-specific marker protein, and/or the like. The protein present at the vesicles may be a glycoprotein. The nucleic acid present at the vesicles may be DNA or RNA, and the RNA may include messenger RNA, microRNA, noncoding RNA, and the like. The biomolecules present at the vesicles may be present inside the vesicles, but preferably, they are present on the surface of the vesicles.

### <Step of Bringing Vesicles into Contact with Capturing Molecules Solidified on Support>

A description will be given of the step of bringing vesicles into contact with capturing molecules solidified on a support. By this step, the vesicles are captured on the support. With the vesicles thus being captured on the support, the vesicles can be concentrated and the detection sensitivity can be enhanced. Further, by washing the support after bringing the vesicles into contact with the capturing molecules, it is possible to remove non-specifically bound vesicles and reduce background noise. By the present invention, even when the amount of vesicles is very small, for example, and even when the number of vesicles contained in the specimen is less than 1×10¹⁰ (for example, not less than 1×10⁶ and less than 1×10¹⁰), for example, it is still possible to quantify biomolecules on the vesicles. Another way of understanding it is that, in an aspect of the present embodiment, the method of analyzing biomolecules present at vesicles is capable of quantifying the density of vesicles (the number of vesicles per unit volume). That is, the method of analyzing biomolecules present at vesicles is capable of quantifying biomolecules on vesicles even when the density of the vesicles is less than 1×10¹⁰/100 µl (for example, not less than 1×10⁶/100 µl and less than 1×10¹⁰/100 µl). Preferably, the capturing molecules are molecules capable of bonding to biomolecules that are known to be present at vesicles. The capturing molecules may be protein (such as antibody and lectin) and/or nucleic acid molecules (such as aptamers, and nucleic acid molecules having a complementary sequence). The capturing molecule may be a molecule capable of bonding to a protein that is expressed on the surface of vesicles, such as, for example, an exosome marker, a microvesicle marker, an apoptotic body marker, and other membrane proteins. The capturing molecule may be a molecule capable of bonding to a disease-specific marker. Preferably, the capturing molecule is a molecule capable of bonding to a marker selected from the group consisting of extracellular vesicle markers and disease-specific markers.

In the case where the capturing molecule is a molecule capable of bonding to an exosome marker and the detection molecule is a molecule capable of bonding to an intended biomolecule, exosomes in the specimen can be captured on the support and, then, expression of the intended biomolecule present at the exosomes can be quantified. When the capturing molecule is a molecule capable of bonding to an extracellular vesicle marker such as a microvesicle marker and an apoptotic body marker and the detection molecule is a molecule capable of bonding to an intended biomolecule, expression of the intended biomolecule present at extracellular vesicles such as a microvesicle marker and an apoptotic body marker can be quantified.

When the capturing molecule is a molecule capable of bonding to a disease-specific marker, vesicles having the disease-specific marker are captured on the support. When at least one of the detection molecules is a molecule capable of bonding to a marker for the vesicles, the amount of the disease-specific marker present at the vesicles can be quantified. When the capturing molecule is a molecule capable of bonding to a disease-specific marker, as compared to when the capturing molecule is a molecule capable of bonding to a vesicle marker, there is a chance that accuracy of detection of the disease-specific marker on the vesicles can be enhanced.

Examples of the exosome marker include Alix, Tsg101, tetraspanin, Flotillin, and the like, and tetraspanin includes CD9, CD63, CD81, and the like. Examples of the microvesicle marker include integrin, selectin, CD40, and the like. Examples of the apoptotic body marker include phosphatidylserine, annexin V, and the like. Examples of the disease-specific marker include Carcinoma Embryonic Antigen (CEA), Prostate specific antigen (PSA), Carbohydrate antigen 19-9 (CA19-9), and the like. The capturing molecule may be an antibody capable of recognizing these proteins. For the purpose of capturing exosomes more selectively, the capturing molecule is preferably selected from the group consisting of anti-CD9 antibody, anti-CD63 antibody, and anti-CD81 antibody.

The support may be a multi-well plate or beads. When a multi-well plate (a 96-well plate, for example) is used as the support, throughput of the analysis can be enhanced. The method for solidifying the capturing molecules on the support is not particularly limited, and the solidification may be achieved by means of chemical bonding, hydrogen bonding, ionic bonding, complex formation, hydrophobic interaction, van der Waals interaction, electrostatic interaction, stereoselective interaction, and/or the like. When the capturing molecule is an antibody, it is possible to use a support on which a linker capable of site-specifically bonding to the Fc domain of the antibody (such as Protein A and Protein G) is fixed and, thereby, solidify the capturing molecules on the support.

For example, when solidification of the capturing molecules is carried out by adding an aqueous solution containing the capturing molecules to a multi-well plate, the concentration of the capturing molecules in the aqueous solution may be from 1 to 10 µg/mL, or may be from 0.1 to 1 µg/mL.

Preferably, the aqueous solution for dissolving the capturing molecules is a buffer. Examples of the buffer include PBS, HEPES buffer, acetate buffer, phosphate buffer, citrate buffer, citrate-phosphate buffer, borate buffer, tartrate buffer, Tris buffer, and the like. The aqueous solution may contain a salt. The concentration of the salt in the aqueous solution is preferably 150 mM or less, more preferably from 10 mM to 150 mM, further preferably from 15 mM to 100 mM. Examples of the salt include sodium chloride (NaCl), potassium chloride (KCl), calcium chloride (CaCl₂), and the like. The pH of the aqueous solution is not particularly limited as long as it allows for stable presence of the capturing molecules in it, and, for example, it is preferably from 6 to 10.

In an aspect of the present embodiment, it is preferable to bring the capturing molecules into contact with the multi-well plate (the support) in an environment at 20°C to 30°C. In other words, it is preferable that the temperature of the aqueous solution be from 20°C to 30°C.

In an aspect of the present embodiment, the duration of contact of the capturing molecules with the support may be from 0.5 hours to 3 hours, for example, or may be from 1 hour to 2 hours.

In another aspect of the present embodiment, the support on which the capturing molecules are solidified may be washed with PBS liquid and/or the like to remove excess capturing molecules. The support on which the capturing molecules are solidified may be subjected to blocking treatment with the use of a blocking agent. Examples of the blocking agent include BSA, Lipidure BL802 (manufactured by NOF Corporation), casein, and the like. The concentration of the blocking agent is preferably from 1 weight% to 10 weight%, more preferably from 2 weight% to 5 weight%.

When the support is a multi-well plate, a specimen containing vesicles may be added to the wells on which the capturing molecules are solidified, for bringing the vesicles into contact with the capturing molecules. When the support is beads, a specimen containing vesicles may be added to and stirred with the beads or a suspension containing the beads on which the capturing molecules are solidified, for bringing the vesicles into contact with the capturing molecules. When the support is a packing material placed in a column, a specimen containing vesicles may be poured into the column. After the vesicles are brought into contact with the capturing molecules, the support may be washed. By washing, unbound vesicles and non-specifically bound vesicles can be removed.

Two or more types of capturing molecules may be solidified on one support (such as one well of a multi-well plate, for example), or one type of capturing molecules may be solidified on one support. In an aspect, one type of capturing molecules may be solidified on one support and another type of capturing molecules may be solidified on another support, and then one specimen may be brought into contact with both capturing molecules to cause capturing of vesicles on both supports.

In the present embodiment, the amount of the capturing molecules solidified on the support is preferably from 0.01 to 2 µg, more preferably from 0.01 to 1 µg, further preferably from 0.01 to 0.5 µg, further more preferably from 0.01 to 0.1 µg.

The vesicles may be derived from cultured cells, cultured tissue, or the resulting culture supernatant, and, preferably, it is derived from a living thing. A specimen derived from a living thing (a biological specimen) may be blood collected from a subject, or may be biological tissue, urine, stool, sweat, saliva, lymph fluid, amniotic fluid, breast milk, pleural fluid, ascitic fluid, cerebrospinal fluid, lacrimal fluid, and/or the like. The blood may be whole blood, and, preferably, it is plasma or serum. The specimen may be diluted with physiological saline, buffer, and/or the like. The biological specimen may be collected as appropriate by a known method which is suitable for the type of the biological specimen. The subject may be a human, or may be an animal that is not a human. When the vesicles are derived from a living thing, the type and amount of biomolecules present at vesicles in the living thing can be measured.

The specimen containing vesicles may be purified or pretreated as appropriate, and may be subjected to filtration, centrifugation, ultracentrifugation, affinity separation, dialysis, sedimentation treatment, and/or the like. The specimen containing vesicles may be a purified vesicle fraction, or may be a purified exosome fraction. When the specimen containing vesicles is a purified exosome fraction, the amount of foreign matter in the specimen can be low, leading to enhanced accuracy of the measurement. Purification of exosomes can be carried out by a known method such as use of a commercially available purification kit, ultracentrifugation, density gradient centrifugation, size exclusion chromatography, filtering treatment, and/or the like. On the other hand, by the method according to the present invention, it is possible to selectively capture and concentrate vesicles from a specimen containing the vesicles, without performing vesicle purification such as ultracentrifugation, to quantify biomolecules contained in the vesicles.

### <Step of Bringing First Detection Molecules Labeled with First Metal Particles into Contact with Vesicles, and Step of Bringing Second Detection Molecules Labeled with Second Metal Particles into Contact with Vesicles>

A description will be given of the step of bringing first detection molecules labeled with first metal particles into contact with vesicles and the step of bringing second detection molecules labeled with second metal particles into contact with vesicles. By these steps, the first metal particles and the second metal particles are bonded to the vesicles. The step of bringing the first detection molecules into contact with the vesicles and the step of bringing the second detection molecules into contact with the vesicles may be carried out sequentially, or may be carried out simultaneously. In other words, the first detection molecules and the second detection molecules may be sequentially brought into contact with the vesicles on the support, or the first detection molecules and the second detection molecules may be mixed together in advance before brought into contact with the vesicles on the support. After the detection molecules are brought into contact with the vesicles, the support may be washed. By washing, excess detection molecules and non-specifically bound detection molecules can be removed.

In the present invention, at least two types of detection molecules are used. The number of types of the detection molecules may be three or more, four or more, five or more, or ten or more, and may be fifty or less. When three or more types of detection molecules are used, the third and later types of detection molecules may be brought into contact with the vesicles at the same time with the first detection molecules or the second detection molecules, or may be brought into contact with the vesicles in a step other than the step of bringing the first detection molecules into contact with the vesicles or the step of bringing the second detection molecules into contact with the vesicles.

In the present invention, either the step of bringing the detection molecules into contact with the vesicles or the step of bringing the vesicles into contact with the capturing molecules solidified on the support may be carried out first. When the step of bringing the vesicles into contact with the capturing molecules is carried out first, a specimen containing the vesicles may be added to the support, and then, to the vesicles thus captured on the support, a solution containing the detection molecules may be added, for example. When the step of bringing the detection molecules into contact with the vesicles is carried out first, a specimen containing the vesicles may be mixed with the detection molecules, and the reaction solution may be added to the support, for example. The first detection molecules and the second detection molecules may be brought into contact with vesicles on one support, or each of the first detection molecules and the second detection molecules may be brought into contact with vesicles on a plurality of supports.

Herein, the first detection molecules and the second detection molecules may be collectively called the detection molecules. The same applies to when three or more types of detection molecules are used. The detection molecules may be molecules capable of bonding to intended biomolecules such as protein, nucleic acid, carbohydrate chain, and/or the like. For example, the detection molecules may include molecules capable of bonding to biomolecules that are known to be present at vesicles, or may include molecules capable of bonding to biomolecules that are not typically present at vesicles or biomolecules that are not constantly present at vesicles. When the biomolecule is a protein, the biomolecule may be a membrane protein, a cell adhesion molecule, a receptor protein, a protein involved in membrane fusion or dissociation, a major histocompatibility complex (MHC) molecule, a heat-shock protein, a disease-specific marker protein, and/or the like. The biomolecule may be a glycoprotein and/or a carbohydrate chain. When the biomolecule is a nucleic acid, the biomolecule may be DNA or RNA, and the RNA may include messenger RNA, microRNA, noncoding RNA, and the like.

The detection molecules may be protein (such as antibody and lectin) and/or nucleic acid molecules (such as aptamers, and nucleic acid molecules having a complementary sequence). At least one of the detection molecules may be a molecule capable of bonding to a biomolecule contained in vesicles, such as, for example, extracellular vesicle markers such as exosome markers, microvesicle markers, and/or apoptotic body markers, other membrane proteins, and carbohydrate chains. At least one of the detection molecules may be a molecule capable of bonding to a disease-specific marker. At least one of the detection molecules (at least one selected from the first detection molecules and the second detection molecules) may be, for example, a molecule capable of bonding to a marker selected from the group consisting of an extracellular vesicle marker and a disease-specific marker. When at least one of the detection molecules is a molecule capable of bonding to an exosome marker, it is possible to identify the type of the vesicles captured on the support, namely, for example, it is possible to identify whether they are exosomes or not. For example, whether the vesicles are exosomes or not can be identified by checking the presence of a plurality of exosome markers. The plurality of exosome markers on the vesicles may be detected by a combination of the capturing molecules and a detection antibody, or may be detected by a combination of at least two types of detection antibodies. When at least one of the detection molecules is a molecule capable of bonding to a disease-specific marker, the disease-specific marker present at the vesicles captured on the support can be quantified.

The first detection molecules and the second detection molecules are different molecules from each other. The first detection molecules and the second detection molecules may be molecules capable of bonding to the same biomolecule, or may be molecules capable of bonding to different biomolecules. One of the detection molecules may be the same as the capturing molecules. At least one of the detection molecules may be capable of bonding to the same molecule as the molecule to which the capturing molecules can bond. At least one of the detection molecules can be bonded to a molecule different from the molecule to which the capture antibody can bond. In an aspect of the present embodiment, the detection molecule may be a molecule different from the capturing molecule.

For example, when the capturing molecule is an antibody against a surface antigen on extracellular vesicles, at least one of the detection molecules may be an antibody against a surface antigen on extracellular vesicles and at least one of the detection molecules may be an antibody against a disease-specific marker protein. For example, when the capturing molecule is an antibody against a disease-specific marker protein, at least one of the detection molecules may be an antibody against a surface antigen on extracellular vesicles.

The plurality of detection molecules are labeled with different metal particles, respectively. In other words, the first metal particles and the second metal particles contain different metallic elements. The amount of the metals can be measured simultaneously, and the amount of the detection molecules associated with the respective metal particles can be evaluated. Examples of the metallic element contained in the metal particles include gold (Au), silver (Ag), platinum (Pt), palladium (Pd), iridium (Ir), rhodium (Rh), ruthenium (Ru), aluminum (Al), copper (Cu), tellurium (Te), bismuth (Bi), lead (Pb), iron (Fe), cerium (Ce), molybdenum (Mo), niobium (Nb), tungsten (W), antimony (Sb), tin (Sn), vanadium (V), manganese (Mn), nickel (Ni), cobalt (Co), zinc (Zn), lanthanum (La), cerium (Ce), praseodymium (Pr), neodymium (Nd), samarium (Sm), europium (Eu), gadolinium (Gd), terbium (Tb), dysprosium (Dy), holmium (Ho), erbium (Er), thulium (Tm), ytterbium (Yb), lutetium (Lu), scandium (Sc), yttrium (Y), titanium (Ti), and the like. From the viewpoint of material stability and easy synthesis, at least one of the detection molecules preferably contains a metallic element selected from gold, platinum, iridium, palladium, silver, and copper, and, preferably, it is composed of one of these metallic elements. In an aspect of the present embodiment, each of the first metal particles and the second metal particles may be an elemental metal, or may be an alloy composed of two or more types of metallic elements.

For example, when the first metal particles are an alloy composed of a metallic element A and a metallic element B and the second metal particles are an alloy composed of a metallic element A and a metallic element C, the amount of the metallic element A corresponds to the total amount of the first detection molecules and the second detection molecules, and the amount of the metallic element B and the metallic element C correspond to the amount of the first detection molecules and the amount of the second detection molecules, respectively. As described below, in the analysis method according to the present embodiment, because it simultaneously quantifies the first metal particles and the second metal particles by ICP mass spectrometry and/or the like, and, thereby, it is possible to simultaneously estimate the total amount of the first detection molecules and the second detection molecules, the amount of the first detection molecules, and the amount of the second detection molecules, and, further, it is also possible to use the total amount of the first detection molecules and the second detection molecules to correct the amount of the first metal particles and the amount of the second metal particles.

The metal particles may be metal nanoparticles, metal nanorods, and/or metal nanoplates. The metal particles are preferably small for not interfering with the bonding between the vesicles and the detection molecules, and the particle size of the metal particle (the size of the largest portion of the particle) is usually from 10 nm to 500 nm, preferably 200 nm or less, more preferably 100 nm or less. When the metal particle is substantially spherical, the diameter may be 500 nm or less, or 200 nm or less, or 100 nm or less. The lower limit to the diameter may be 10 nm or more. When the metal particle is a metal nanorod, the length of the minor axis may be 100 nm or less, or 50 nm or less, or 10 nm or less, and the length of the major axis may be 500 nm or less, or 200 nm or less, or 100 nm or less. The lower limit to the length of the minor axis may be 2 nm or more. The lower limit to the length of the major axis may be 5 nm or more. When the metal particle is a metal nanoplate, the thickness may be 100 nm or less, or 50 nm or less, or 30 nm or less, and the maximum length of the flat plane may be 500 nm or less, or 200 nm or less, or 100 nm or less. The lower limit to the thickness may be 4 nm or more. The lower limit to the maximum length of the flat plane may be 10 nm or more. The size of the metal particles may be measured by scanning electron microscope (SEM) examination, scanning transmission electron microscope (STEM) examination, or transmission electron microscope (TEM) examination, for example. Examples of the apparatus include an ultrahigh-resolution analytical scanning electron microscope SU-70 (manufactured by Hitachi, Ltd.). The longest diameter may be measured with a dynamic light scattering particle size distribution measurement apparatus (DLS). Examples of the apparatus include a zeta-potential & particle size & molecular weight analyzer ELSZ-2000ZS (manufactured by Otsuka Electronics Co., Ltd.). Examples of other apparatuses for measuring the longest diameter include a laser diffraction particle size distribution measurement apparatus SALD-7500nano (manufactured by Shimadzu Corporation) and a nanoparticle analysis system NanoSight LM10 (Malvern Panalytical). When it is intended to measure the longest diameter of anisotropic gold nanoparticles such as metal nanorods and metal nanoplates in an SEM photograph, an STEM photograph, or a TEM photograph, the longest diameter of 125 randomly-selected anisotropic gold nanoparticles may be measured, and from the resulting data of 125 longest diameters in total, the upper 10% and the lower 10% may be removed to prepare data of 100 longest diameters, which may be then averaged to obtain the longest diameter.

In the present embodiment, the surface of the metal particles may be covered with a surfactant. This provides stability to the metal particles in the aqueous solution. In an aspect of the present embodiment, from the viewpoint of enhancing the stability of the metal particles in the aqueous solution, the aqueous solution may contain a dispersant. Examples of the dispersant include sodium citrate.

In the present embodiment, the method of labeling the detection molecules with the metal particles is not particularly limited, and a known method may be employed. Examples thereof include chemical modification with a functional group such as maleimide, physical adsorption, and the like. Alternatively, a commercially available detection molecules labeled with metal particles may be purchased.

In the present embodiment, preferably, each of the first detection molecules and the second detection molecules are brought into contact with the vesicles in an aqueous solution containing a salt. The concentration of the salt in the aqueous solution is preferably 150 mM or less, more preferably from 15 mM to 150 mM, further preferably from 20 mM to 150 mM, further more preferably from 20 mM to 100 mM. Examples of the above-mentioned salt include sodium chloride (NaCl), potassium chloride (KCl), calcium chloride (CaCl₂), and the like. In this case, as the concentration of the detection molecules, absorbance at a maximum absorption wavelength is measured for 100 µL of the solution in a 96-well plate with a plate reader (Infinite M-200 pro, manufactured by TECAN), and it is preferably from 0.01 to 2, more preferably from 0.5 to 1.5. When the maximum absorption wavelength is not present, absorbance at an absorption wavelength of 550 nm is used.

In an aspect of the present embodiment, the aqueous solution containing a salt is preferably a buffer. Examples of the buffer include PBS, HEPES buffer, acetate buffer, acetate-phosphate buffer, citrate buffer, citrate-phosphate buffer, borate buffer, tartrate buffer, Tris buffer, and the like. The pH of the aqueous solution containing a salt is not particularly limited as long as it allows for stable presence of the detection molecules in it, and, for example, it is more preferably from 6 to 10.

In addition, from the viewpoint of stabilizing the detection molecules, the aqueous solution containing a salt may further contain a blocking agent. Examples of the blocking agent include BSA, Lipidure BL802 (manufactured by NOF Corporation), casein, and the like. The concentration of the blocking agent is preferably from 1 weight% to 10 weight%, more preferably from 2 weight% to 5 weight%.

In another aspect of the present embodiment, it is preferable to bring each of the first detection molecules and the second detection molecules into contact with the vesicles in an environment at 20°C to 30°C. In other words, it is preferable that the temperature of the aqueous solution containing a salt be from 20°C to 30°C.

In the present embodiment, the duration of contact of the detection molecules with the vesicles is not particularly limited as long as the interaction of the detection molecules with the vesicles reaches equilibrium, and, for example, it may be from 0.5 hours to 3 hours, or may be from 1 hour to 2 hours.

### <Step of Quantifying First Metal Particles and Second Metal Particles Bonded to Vesicles by Inductively Coupled Plasma Mass Spectrometry or ICP Emission Spectroscopy>

A description will be given of the step of quantifying the first metal particles and the second metal particles bonded to the vesicles by inductively coupled plasma mass spectrometry (ICP-MS) or ICP emission spectroscopy (ICP-AES/ICP-OES). The metal particles bonded to the vesicles via the detection molecules are firstly dissolved and extracted with acid such as aqua regia (a mixed liquid of concentrated nitric acid and concentrated hydrochloric acid), nitric acid, hydrochloric acid, sulfuric acid, and/or the like. A peeling liquid may be used to separate the metal particles from the support, and the metal particles contained in the peeling liquid may be dissolved with acid. The concentration of the aqua regia ((concentrated hydrochloric acid):(concentrated nitric acid)=3: 1) may be 50% or more. The temperature for dissolving the metal particles may be 25°C or more, for example, and the reaction time may be from 10 minutes to 24 hours. From the viewpoint of detecting the metal particles with high sensitivity, it is preferable to dissolve the metal particles with aqua regia.

When the first detection molecules and the second detection molecules are bonded to one support, the acid treatment yields a solution containing a plurality of metallic elements. When the first detection molecules are bonded to one support and the second detection molecules are bonded to another support, the solutions containing metallic elements resulting from the acid treatment may be mixed together to obtain a solution containing a plurality of metallic elements.

The solution containing a plurality of metallic elements is introduced into an ICP-MS apparatus or an ICP-AES apparatus. In the case of ICP-MS, the metallic elements in the solution are ionized by inductively coupled plasma and separated by an electric field, a magnetic field, and/or the like, and thereby the concentrations of the elements are quantified. In the case of ICP-AES, from the wavelength and intensity of light emitted from the elements excited by plasma, the concentrations of the elements are quantified. The inductively coupled plasma may be argon gas plasma.

The amount of metal ions thus quantified correlates with the amount of biomolecules present at the vesicles. ICP-MS or ICP-AES can simultaneously quantify the amount of a plurality of metal ions, so it can simultaneously quantify a plurality of biomolecules present at the vesicles with high sensitivity.

Examples of the ICP-MS apparatus include ICPMS-2030 (manufactured by Shimadzu Corporation) and the like. Examples of the ICP-AES apparatus include ICPE-2030 (manufactured by Shimadzu Corporation).

### [Diagnosis Assisting Method]

A diagnosis assisting method according to an aspect of the present invention comprises a step of providing information for disease diagnosis, based on the type and amount of biomolecules present at vesicles obtained by the above-mentioned analysis method.

The diagnosis assisting method according to the present invention makes it possible to provide information for disease diagnosis in an easy and simple manner. The diagnosis assisting method according to the present invention does not include a step of determination by a doctor, so it does not fall under the category of a method of diagnosis of a human. The diagnosis assisting method according to the present invention is capable of providing information for disease diagnosis in a non-invasive manner.

The diagnosis assisting method according to the present invention may comprise a step of providing information for disease diagnosis, based on the above-mentioned method of analyzing biomolecules present at vesicles or based on the type and amount of biomolecules present at vesicles obtained by the above-mentioned analysis method.

Specific biomolecules present at vesicles obtained from a biological specimen, such as, for example, extracellular vesicle markers, disease-specific markers, and/or a combination of these may be used as a disease diagnostic marker. The disease-specific marker may be a biomolecule, including a nucleic acid such as miRNA, lipid, and protein. Examples of the disease include cancers such as lung cancer, colorectal cancer, pancreatic cancer, cervical cancer, breast cancer, and glioblastoma, lifestyle-related diseases, neuropsychiatric diseases, immune diseases and allergic diseases, infectious diseases, and the like.

### [Examples]

In the following, a more detailed description will be given of the present invention by way of examples, but these are not intended to limit the scope of the present invention.

### (Experiment 1)

Medium supernatant obtained from incubation of cultured cells (MCF7) was filtrated through a filter (0.22 µm), and the resulting filtrate was subjected to ultracentrifugation (100000 g, 70 min) treatment to prepare an exosome fraction. The exosome fraction thus obtained was suspended in PBS to prepare specimens that contained 1.0×10¹⁰, 1.0×10⁹, 1.0×10⁸, 1.0×10⁷, 1.0×10⁶, or 0 (blank) exosome particles, respectively. The number of particles was detected with the use of NanoSight (Quantum Design Japan, Inc.). The following were prepared: detection molecules that were composed of anti-CD9 antibody metal-labeled with gold (Au) particles (particle size, 40 nm) (Au-CD9 Ab); and detection molecules that were composed of anti-CD81 antibody metal-labeled with palladium (Pd) particles (particle size, 100 nm) (Pd-CD81 Ab).

To wells on which anti-CD9 antibody was solidified, the specimen containing exosomes was added, to let the exosomes become captured on the support. Each of a Au-CD9 Ab solution and a Pd-CD81 Ab solution was added to the wells, and the surface of the captured exosomes was subjected to antibody staining. Excess antibody was removed by washing. Aqua regia was added to the wells to elute metal particles that were bonded to the exosomes via the antibody. The solution containing the metal particles was subjected to ICP-MS ("ICPMS-2030" manufactured by Shimadzu Corporation) to quantify Au ions and Pd ions. In this way, surface antigens on the exosomes can be quantified.

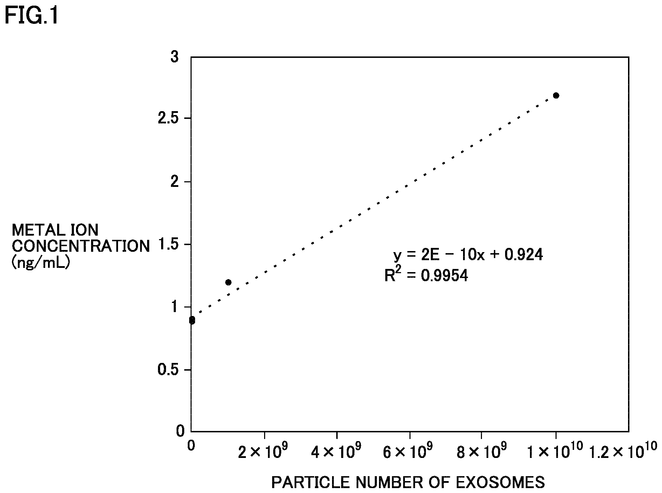

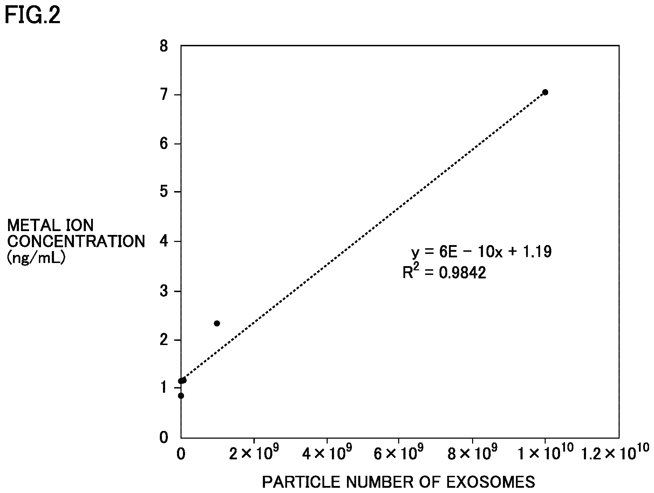

The amount of Au ions for each exosome particle number was quantified to plot the amount of Au ions relative to the exosome particle number, and, as a result, it was found that the amount of Au ions was increased with a strong correlation to the exosome particle number (Fig. 1). Similarly, the amount of Pd ions for each exosome particle number was quantified to plot the amount of Pd ions relative to the exosome particle number, and, as a result, it was found that the amount of Pd ions was increased with a strong correlation to the exosome particle number (Fig. 2). When the exosome particle number was less than 10¹⁰, surface antigens were quantified with high sensitivity.

This method demonstrates that it is possible to quantify biomolecules at vesicles with the use of various antibodies (detection molecules) labeled with various metal particles. The method according to the present invention suggests that it is possible to simultaneously quantify intended biomolecules at vesicles in a specimen.

### (Experiment 2)

Medium supernatant obtained from incubation of cultured cells (MCF7) was filtrated through a filter (0.22 µm), and the resulting filtrate was subjected to ultracentrifugation (100000 g, 70 min) treatment to prepare an exosome fraction. The exosome fraction thus obtained was suspended in PBS to prepare specimens that contained 1.0×10¹⁰, 1.0×10⁹, 1.0×10⁸, 1.0×10⁷, 1.0×10⁶, or 0 (blank) exosome particles, respectively, each in 100 µl. The number of particles was measured with the use of NanoSight (Quantum Design Japan, Inc.). The following were prepared: detection molecules that were composed of anti-CD9 antibody metal-labeled with gold (Au) particles (particle size, 40 nm) (Au-CD9 Ab) (concentration, 0.1 in terms of absorbance at 530 nm); detection molecules that were composed of anti-CD63 antibody metal-labeled with platinum (Pt) particles (particle size, 30 nm) (Pt-CD63 Ab) (concentration, 0.1 in term of absorbance at 500 nm); and detection molecules that were composed of anti-CD81 antibody metal-labeled with palladium (Pd) particles (particle size, 100 nm) (Pd-CD81 Ab) (concentration, 0.1 in term of absorbance at 500 nm).

After the above-described preparation, anti-CD9 antibody (capturing molecules) was solidified on a 96-well plate according to the procedure described below. Firstly, a solution containing anti-CD9 antibody (concentration, 0.1 in terms of absorbance at 530 nm) was added in an amount of 100 µl per well, followed by incubation at 4°C for 12 hours. Then, a blocking agent was added to each well in an amount of 200 µl per well, followed by incubation at room temperature for 2 hours. Subsequently, to each well on which the anti-CD9 antibody was solidified, 100 µl of the specimen containing exosomes was added, to let the exosomes become captured on the support. A mixed liquid of equal parts of a Au-CD9 Ab solution, a Pt-CD63 Ab solution, and a Pd-CD81 Ab solution was added in an amount of 100 µl to each well, and the surface of the captured exosomes was subjected to antibody staining. Each metal-labeled antibody described above was used after it was suspended in an aqueous solution containing 10-mM HEPES (pH 7.5), 1% BSA, 150-mM NaCl, and 0.05% Lipidure BL802 (the aqueous solution is expressed as "150-mM NaCl"), or in an aqueous solution containing 10-mM HEPES (pH 7.5), 1% BSA, 20-mM NaCl, and 0.05% Lipidure BL802 (the aqueous solution is expressed as "20-mM NaCl"). After the reaction with the metal-labeled antibody, excess antibody was removed by washing with a washing liquid (10-mM HEPES (pH 7.5), 20-mM NaCl aqueous solution). Aqua regia was added to each well in an amount of 300 µl to elute metal particles that were bonded to the exosomes via the antibody. The resulting solution containing the metal particles was 18-fold diluted with water and subjected to ICP-MS ("ICPMS-2030" manufactured by Shimadzu Corporation) to quantify Au ions, Pt ions, and Pd ions. In this way, surface antigens on the exosomes were quantified.

The amount of Au, Pt, and Pd ions for each exosome particle number was quantified, and the ratio of the amount of detected ions for each exosome particle number to the amount of detected ions in the blank (the ratio S/N) was calculated (Table 1). As for exosome analysis with 10¹⁰ and 10⁹ particles, the ratio S/N for the antibody reaction in 20-mM NaCl aqueous solution was higher than that for the reaction in 150-mM NaCl aqueous solution (Table 1). Usually, salts are used for stabilizing the antibody reaction and the metal particle dispersibility. Hence, it is indicated that the lower the salt concentration of the aqueous solution used for antibody reaction is, the better the resulting data obtainable in exosome mass spectrometry can become.

**[Table 1]**

| (1) 150 mM NaCl | | | | | | | | | | | | |
|---|---|---|---|---|---|---|---|---|---|---|---|---|
| EVs (Number) | Au | | | | Pt | | | | Pd | | | |
| | AVE (ng) | SE (ng) | CV | S/N | AVE (ng) | SE (ng) | CV | S/N | AVE (ng) | SE (ng) | CV | S/N |
| 0 | 0.9 | 0.1 | 0.07 | | 8.9 | 1.8 | 0.20 | | 298 | 67 | 0.23 | |
| 1×10⁶ | 1.1 | 0.1 | 0.09 | 1.2 | 11.3 | 1.7 | 0.15 | 1.3 | 400 | 81 | 0.20 | 1.3 |
| 1×10⁷ | 1.1 | 0.2 | 0.21 | 1.3 | 9.6 | 2.0 | 0.21 | 1.1 | 340 | 54 | 0.16 | 1.1 |
| 1×10⁸ | 4.3 | 2.4 | 0.57 | 4.8 | 15.5 | 5.0 | 0.32 | 1.7 | 418 | 26 | 0.06 | 1.4 |
| 1×10⁹ | 13.0 | 0.5 | 0.04 | 14.6 | 15.5 | 1.2 | 0.07 | 1.7 | 449 | 40 | 0.09 | 1.5 |
| 1×10¹⁰ | 116.3 | 7.2 | 0.06 | 130.2 | 50.1 | 3.4 | 0.07 | 5.6 | 494 | 79 | 0.16 | 1.7 |

| | | | | | | | | | | | | |
|---|---|---|---|---|---|---|---|---|---|---|---|---|
| AVE: Average amount of metal particles, SE: Standard deviation, CV: Coefficient of variation | | | | | | | | | | | | |

### (2) 20 mM NaCl

| EVs (Number) | Au | | | | Pt | | | | Pd | | | |
|---|---|---|---|---|---|---|---|---|---|---|---|---|
| | AVE (ng) | SE (ng) | CV | S/N | AVE (ng) | SE (ng) | CV | S/N | AVE (ng) | SE (ng) | CV | S/N |
| 0 | 0.04 | 0.01 | 0.33 | | 0.32 | 0.02 | 0.05 | | 0.70 | 0.04 | 0.05 | |
| 1×10⁶ | 0.03 | 0.01 | 0.27 | 0.9 | 0.27 | 0.04 | 0.14 | 0.8 | 0.94 | 0.08 | 0.08 | 1.4 |
| 1×10⁷ | 0.22 | 0.25 | 1.15 | 5.4 | 0.99 | 0.53 | 0.54 | 3.1 | 1.41 | 0.56 | 0.40 | 2.0 |
| 1×10⁸ | 0.23 | 0.10 | 0.44 | 5.6 | 0.46 | 0.14 | 0.30 | 1.4 | 1.12 | 0.22 | 0.20 | 1.6 |
| 1×10⁹ | 1.77 | 0.12 | 0.07 | 43.9 | 1.57 | 0.34 | 0.21 | 4.9 | 3.76 | 0.85 | 0.23 | 5.4 |
| 1×10¹⁰ | 27.37 | 2.17 | 0.08 | 677.0 | 11.58 | 1.17 | 0.10 | 36.0 | 30.53 | 4.01 | 0.13 | 43.8 |

| | | | | | | | | | | | | |
|---|---|---|---|---|---|---|---|---|---|---|---|---|
| AVE: Average amount of metal particles, SE: Standard deviation, CV: Coefficient of variation | | | | | | | | | | | | |

### (Experiment 3)

Medium supernatant obtained from incubation of cultured cells (MCF7) was filtrated through a filter (0.22 µm), and the resulting filtrate was subjected to ultracentrifugation (100000 g, 70 min) treatment to prepare an exosome fraction. The exosome fraction thus obtained was suspended in PBS to prepare specimens that contained 1.0×10¹⁰, 1.0×10⁹, 1.0×10⁸, 1.0×10⁷, 1.0×10⁶, or 0 (blank) exosome particles (particles), respectively, each in 100 µl. The number of particles was measured with the use of NanoSight (Quantum Design Japan, Inc.). The following were prepared: detection molecules that were composed of anti-CD9 antibody metal-labeled with gold (Au) particles (particle size, 40 nm) (Au-CD9 Ab) (concentration, 0.1 in terms of absorbance at 530 nm); detection molecules that were composed of anti-CD63 antibody metal-labeled with platinum (Pt) particles (particle size, 30 nm) (Pt-CD63 Ab) (concentration, 0.1 in term of absorbance at 500 nm); and detection molecules that were composed of anti-CD81 antibody metal-labeled with palladium (Pd) particles (particle size, 100 nm) (Pd-CD81 Ab) (concentration, 0.1 in term of absorbance at 500 nm).

After the above-described preparation, by the same procedure as in (Experiment 2), anti-CD9 antibody (capturing molecules) was solidified on a 96-well plate. Subsequently, to each well on which the anti-CD9 antibody was solidified, 100 µl of the specimen containing exosomes was added, to let the exosomes become captured on the support. A mixed liquid of equal parts of a Au-CD9 Ab solution, a Pt-CD63 Ab solution, and a Pd-CD81 Ab solution was added in an amount of 100 µl to each well, and the surface of the captured exosomes was subjected to antibody staining. Each metal-labeled antibody described above was used after it was suspended in an aqueous solution containing 10-mM HEPES (pH 7.5), 1% BSA, 20-mM NaCl, and 0.05% Lipidure BL802. After the reaction with the metal-labeled antibody, excess antibody was removed by washing with a washing liquid (10-mM HEPES (pH 7.5), 20-mM NaCl aqueous solution). Aqua regia was added to each well in an amount of 300 µl to elute metal particles that were bonded to the exosomes via the antibody. The resulting solution containing the metal particles was 18-fold diluted with water and subjected to ICP-MS ("ICPMS-2030" manufactured by Shimadzu Corporation) to quantify Au, Pt, and Pd ions. In this way, surface antigens on the exosomes were quantified.

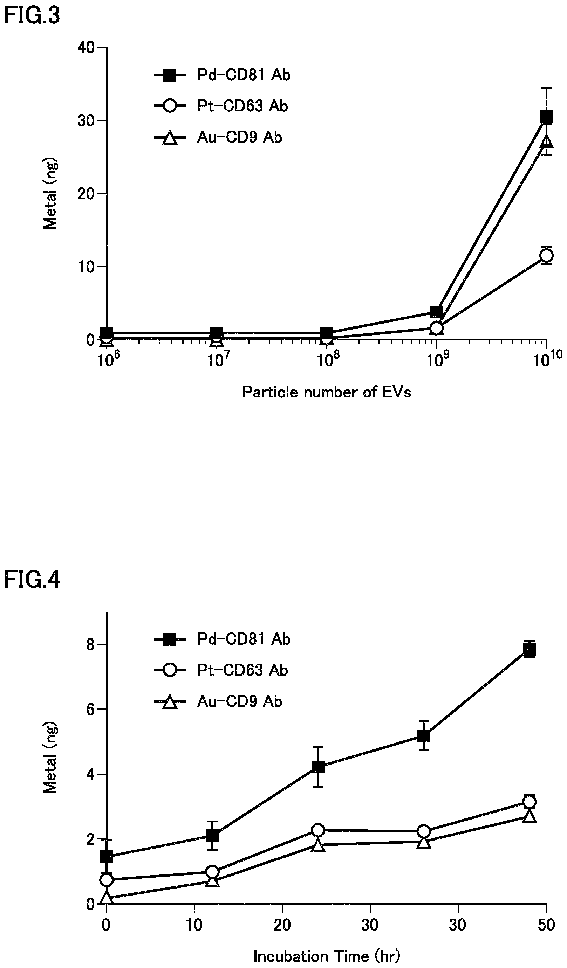

The amount of Au, Pt, and Pd ions for each exosome particle number was quantified to plot the amount of the three types of ions relative to the exosome particle number, and, as a result, it was found that the amount of all the three types of metal ions was increased with a strong correlation to the exosome particle number (with a coefficient of determination of R2>0.99) (Fig. 3).

### (Experiment 4)

Cultured cells (MCF7) were incubated for up to 48 hours, and medium supernatant was collected at different points in time (0, 12, 24, 36, and 48 hours after the start of incubation), which was then filtrated through a filter (0.22 µm) to prepare a sample for analysis. The following were prepared: detection molecules that were composed of anti-CD9 antibody metal-labeled with gold (Au) particles (particle size, 40 nm) (Au-CD9 Ab) (concentration, 0.1 in terms of absorbance at 530 nm); detection molecules that were composed of anti-CD63 antibody metal-labeled with platinum (Pt) particles (particle size, 30 nm) (Pt-CD63 Ab) (concentration, 0.1 in term of absorbance at 500 nm); and detection molecules that were composed of anti-CD81 antibody metal-labeled with palladium (Pd) particles (particle size, 100 nm) (Pd-CD81 Ab) (concentration, 0.1 in term of absorbance at 500 nm).

After the above-described preparation, by the same procedure as in (Experiment 2), anti-CD9 antibody (capturing molecules) was solidified on a 96-well plate. Subsequently, to each well on which the anti-CD9 antibody was solidified, 100 µl of the medium supernatant specimen obtained above was added, to let the exosomes become captured on the support. A mixed liquid of equal parts of Au-CD9 Ab, Pt-CD63 Ab, and Pd-CD81 Ab was added in an amount of 100 µl to each well, and the surface of the captured exosomes was subjected to antibody staining. Each metal-labeled antibody described above was used after it was suspended in an aqueous solution containing 10-mM HEPES (pH 7.5), 1% BSA, 20-mM NaCl, and 0.05% Lipidure BL802. After the reaction with the metal-labeled antibody, excess antibody was removed by washing. Aqua regia was added to each well in an amount of 300 µl to elute metal particles that were bonded to the exosomes via the antibody. The solution containing the metal particles was subjected to ICP-MS ("ICPMS-2030" manufactured by Shimadzu Corporation) to quantify Au, Pt, and Pd ions. In this way, surface antigens on the exosomes were quantified.

The sample that was obtained in the above-described manner by pretreatment of medium supernatant collected at different incubation time was subjected to quantification of the amount of Au, Pt, and Pd ions to plot the amount of the three types of ions in the sample, and, as a result, it was found that the amount of all the three types of metal ions was increased as the incubation time increased (Fig. 4). Hence, it is suggested that the present method is capable of monitoring the amount of exosome secretion in the course of cell incubation.

### (Experiment 5)

Tween-20 was added to a serum specimen (10 µL, 50 µL, 100 µL) in a final concentration of 0.05%, to prepare a sample, which was to be subjected to analysis. The following were prepared: detection molecules that were composed of anti-CD9 antibody metal-labeled with gold (Au) particles (particle size, 40 nm) (Au-CD9 Ab) (concentration, 0.1 in terms of absorbance at 530 nm); detection molecules that were composed of anti-CD63 antibody metal-labeled with platinum (Pt) particles (particle size, 30 nm) (Pt-CD63 Ab) (concentration, 0.1 in term of absorbance at 500 nm); and detection molecules that were composed of anti-CD81 antibody metal-labeled with palladium (Pd) particles (particle size, 100 nm) (Pd-CD81 Ab) (concentration, 0.1 in term of absorbance at 500 nm).

After the above-described preparation, by the same procedure as in (Experiment 2), anti-CD9 antibody (capturing molecules) was solidified on a 96-well plate. Subsequently, to each well on which the anti-CD9 antibody was solidified, 10 µL, 50 µL, or 100 µL of the above-mentioned serum specimen was added, to let the exosomes become captured on the support. A mixed liquid of equal parts of a Au-CD9 Ab solution, a Pt-CD63 Ab solution, and a Pd-CD81 Ab solution was added in an amount of 100 µl to each well, and the surface of the captured exosomes was subjected to antibody staining. Each metal-labeled antibody described above was used after it was suspended in an aqueous solution containing 10-mM HEPES (pH 7.5), 1% BSA, 20-mM NaCl, and 0.05% Lipidure BL802. After the reaction with the metal-labeled antibody, excess antibody was removed by washing with a washing liquid (10-mM HEPES (pH 7.5), 20-mM NaCl aqueous solution). Aqua regia was added to each well in an amount of 300 µl to elute metal particles that were bonded to the exosomes via the antibody. The resulting solution containing the metal particles was 18-fold diluted with water and subjected to ICP-MS ("ICPMS-2030" manufactured by Shimadzu Corporation) to quantify Au, Pt, and Pd ions. In this way, surface antigens on the exosomes were quantified.

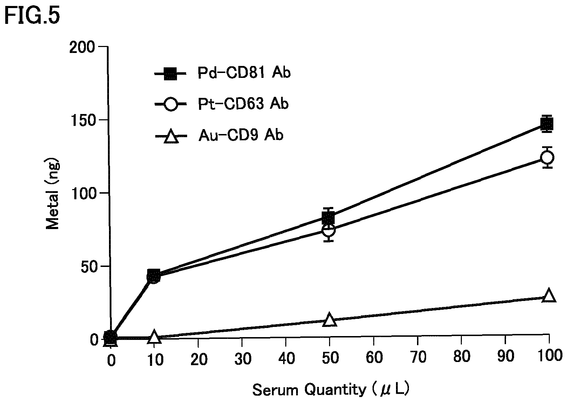

The sample that was obtained in the above-described manner by pretreatment of different amounts of serum specimen was subjected to quantification of the amount of Au, Pt, and Pd ions to plot the amount of the three types of ions in the sample, and, as a result, it was found that the amount of all the three types of metal ions was increased as the amount of the analysis sample increased (Fig. 5). Hence, it is suggested that it is possible to apply the present method to a serum specimen in an amount of at least 10 µL.

### [Aspects]

As will be appreciated by those skilled in the art, the above-described example embodiments and Examples are specific examples of the below aspects.

### (Item 1)

A method of analyzing a biomolecule present at a vesicle according to an aspect comprises:
bringing a vesicle into contact with a capturing molecule solidified on a support;
bringing a first detection molecule labeled with a first metal particle into contact with the vesicle;
bringing a second detection molecule labeled with a second metal particle into contact with the vesicle; and
quantifying the first metal particle and the second metal particle bonded to the vesicle by inductively coupled plasma (ICP) mass spectrometry or ICP emission spectroscopy.

With the analysis method according to Item 1, it is possible to simultaneously quantify a plurality of biomolecules present at vesicles.

### (Item 2)

In the analysis method according to Item 1, the first metal particle and the second metal particle contain different metallic elements.

With the analysis method according to Item 2, it is possible to measure the amount of respective metals.

### (Item 3)

In the analysis method according to Item 1 or Item 2, each of the first detection molecule and the second detection molecule is brought into contact with the vesicle in an aqueous solution containing a salt. With the analysis method according to Item 3, it is possible to accurately quantify a plurality of biomolecules present at vesicles.

### (Item 4)

In the analysis method according to any one of Item 1 to Item 3, the capturing molecule is a molecule capable of bonding to a marker selected from the group consisting of an extracellular vesicle marker and a disease-specific marker.

With the analysis method according to Item 4, when the capturing molecule is capable of bonding to an extracellular vesicle marker, it is possible to capture an extracellular vesicle on the support. When the capturing molecule is capable of bonding to a disease-specific marker, a vesicle that contains the disease-specific marker is captured on the support.

### (Item 5)

In the analysis method according to Item 4, the capturing molecule is selected from the group consisting of an anti-CD9 antibody, an anti-CD63 antibody, and an anti-CD81 antibody.

With the analysis method according to Item 5, it is possible to capture exosomes on the support.

### (Item 6)

In the analysis method according to any one of Item 1 to Item 5, at least one selected from the first detection molecule and the second detection molecule is a molecule capable of bonding to a biomolecule contained in a vesicle.

With the analysis method according to Item 6, it is possible to detect and quantify an intended molecule contained in vesicles.

### (Item 7)

In the analysis method according to Item 6, at least one selected from the first detection molecule and the second detection molecule is a molecule capable of bonding to a marker selected from the group consisting of an extracellular vesicle marker and a disease-specific marker.

With the analysis method according to Item 7, it is possible to identify the type of vesicles. Also, it is possible to quantify expression of a disease-specific marker at vesicles.

### (Item 8)

In the analysis method according to any one of Item 1 to Item 7, at least one selected from the first metal particle and the second metal particle contains a metallic element selected from the group consisting of gold, platinum, iridium, palladium, silver, and copper.

The metallic element according to Item 8 has excellent stability and easy to synthesize.

### (Item 9)

In the analysis method according to any one of Item 1 to Item 8, each of a particle size of the first metal particle and a particle size of the second metal particle is 500 nm or less.

With the analysis method according to Item 9, bonding between vesicles and detection molecules tends not to be interfered with.

### (Item 10)

In the analysis method according to any one of Item 1 to Item 9, the vesicle is derived from a living thing.

With the analysis method according to Item 10, it is possible to measure the type and amount of biomolecules present at vesicles derived from a living thing.

### (Item 11)

In the analysis method according to any one of Item 1 to Item 10, the vesicle is an exosome.

The analysis method according to Item 11 is capable of quantifying biomolecules contained in exosomes and may be used for detection and diagnosis of various diseases including cancer and lifestyle-related diseases.

### (Item 12)

In the analysis method according to any one of Item 1 to Item 11, the support is a multi-well plate.

With the analysis method according to Item 12, it is possible to enhance the throughput of analysis.

### (Item 13)

The analysis method according to any one of Item 1 to Item 12 comprises dissolving at least one of the first metal particle and the second metal particle bonded to the vesicle, with aqua regia.

With the analysis method according to Item 13, it is possible to detect metal particles with high sensitivity and it is possible to enhance sensitivity of detection of biomolecules contained in vesicles.

### (Item 14)

A diagnosis assisting method according to an aspect comprises providing information for disease diagnosis, based on a type and amount of a biomolecule present at a vesicle obtained by the analysis method according to Item 1 to Item 13.

With the diagnosis assisting method according to Item 14, it is possible to provide information for disease diagnosis in a non-invasive manner.

## Claims

1. A method of analyzing a biomolecule present at a vesicle, the method comprising:
bringing a vesicle into contact with a capturing molecule solidified on a support;
bringing a first detection molecule labeled with a first metal particle into contact with the vesicle;
bringing a second detection molecule labeled with a second metal particle into contact with the vesicle; and
quantifying the first metal particle and the second metal particle bonded to the vesicle by inductively coupled plasma (ICP) mass spectrometry or ICP emission spectroscopy.

2. The method according to claim 1, wherein the first metal particle and the second metal particle contain different metallic elements.

3. The method according to claim 1 or 2, wherein each of the first detection molecule and the second detection molecule is brought into contact with the vesicle in an aqueous solution containing a salt.

4. The method according to claim 1 or 2, wherein the capturing molecule is a molecule capable of bonding to a marker selected from the group consisting of an extracellular vesicle marker and a disease-specific marker.

5. The method according to claim 4, wherein the capturing molecule is selected from the group consisting of an anti-CD9 antibody, an anti-CD63 antibody, and an anti-CD81 antibody.

6. The method according to claim 1 or 2, wherein at least one selected from the first detection molecule and the second detection molecule is a molecule capable of bonding to a biomolecule contained in a vesicle.

7. The method according to claim 6, wherein at least one selected from the first detection molecule and the second detection molecule is a molecule capable of bonding to a marker selected from the group consisting of an extracellular vesicle marker and a disease-specific marker.

8. The method according to claim 1 or 2, wherein at least one selected from the first metal particle and the second metal particle contains a metallic element selected from the group consisting of gold, platinum, iridium, palladium, silver, and copper.

9. The method according to claim 1 or 2, wherein each of a particle size of the first metal particle and a particle size of the second metal particle is 500 nm or less.

10. The method according to claim 1 or 2, wherein the vesicle is derived from a living thing.

11. The method according to claim 1 or 2, wherein the vesicle is an exosome.

12. The method according to claim 1 or 2, wherein the support is a multi-well plate.

13. The method according to claim 1 or 2, comprising dissolving at least one of the first metal particle and the second metal particle bonded to the vesicle, with aqua regia.

14. A diagnosis assisting method, comprising providing information for disease diagnosis, based on a type and amount of a biomolecule present at a vesicle obtained by the method according to claim 1 or 2.
